Europäisches Patentamt

European Patent Office

Office européen des brevets

(19)

(11) **EP 1 683 874 A2**

(12) **EUROPEAN PATENT APPLICATION**

(43) Date of publication:
26.07.2006 Bulletin 2006/30

(51) Int Cl.:
*C12Q 1/68* (2006.01)   *G01N 33/53* (2006.01)
*C12N 15/00* (2006.01)   *C07H 21/02* (2006.01)
*G01N 33/50* (2006.01)

(21) Application number: 06112291.7

(22) Date of filing: 29.08.2001

(84) Designated Contracting States:
**AT BE CH CY DE DK ES FI FR GB GR IE IT LI LU MC NL PT SE TR**
Designated Extension States:
**AL LT LV MK RO SI**

(30) Priority: **29.08.2000 US 228420 P**
**20.07.2001 US 306457 P**

(62) Document number(s) of the earlier application(s) in accordance with Art. 76 EPC:
**01963353.6 / 1 313 883**

(71) Applicant: **YEDA RESEARCH AND DEVELOPMENT CO. LTD.**
**Rehovot 76100 (IL)**

(72) Inventors:
  • **Geiger, Benjamin**
    **76352 Rehovot (IL)**

  • **Kam, Zvi**
    **64365 Tel Aviv (IL)**
  • **Zamir, Eli**
    **62381 Tel Aviv (IL)**
  • **Bershadsky, Alexander**
    **76100 Rehovot (IL)**
  • **Shtutman, Michael**
    **76284 Rehovot (IL)**
  • **Ben-Ze'ev, Avri**
    **76468 Rehovot (IL)**

(74) Representative: **Schmitz, Jean-Marie et al**
**Dennemeyer & Associates S.A.**
**P.O. Box 1502**
**1015 Luxembourg (LU)**

Remarks:
This application was filed on 06 - 04 - 2006 as a divisional application to the application mentioned under INID code 62.

(54) **Methods of isolating genes encoding proteins of specific function and of screening for pharmaceutically active agents**

(57)   The present invention relates to a method of identifying at least one agent capable of at least partially reversing an abnormal cellular phenotype, the method comprising (a) exposing the cell characterized by the abnormal phenotype to the at least one agent; and (b) monitoring a change in at least one cellular constituent being associated with the abnormal cellular phenotype, said change being indicative of at least a partial phenotype reversal, to thereby determine the capability of the at least one agent in at least partially reversing the abnormal cellular phenotype, as well as to a method of quantifying a co-localization of a plurality of distinguishable molecules in a cell, the method comprising the steps of (a) acquiring an image of the cell for each of said plurality of distinguishable molecules so as to obtain a plurality of images of said cell, each individual image of said plurality of images presenting a distribution of one of said plurality of distinguishable molecules in the cell; and (b) calculating a correlation coefficient for at least one pair of said individual images, thereby quantifying the co-localization of at least a pair of said distinguishable molecules in the cell.

EP 1 683 874 A2

**Description**

FIELD AND BACKGROUND OF THE INVENTION

The present invention relates to methods of isolating genes encoding proteins of specific function, such as, proteins which are localized to specific subcellular organelles or structures, proteins involved in the formation, organization and/or maintenance of specific subcellular organelles or structures and proteins which bind other proteins. The present invention further relates to a method of screening for pharmaceutically active agents, capable of at least partially reversing an abnormal cellular phenotype.

Due to the extensive amount of information generated by genome-wide sequencing, the entire set of gene products in an organism can now be predicted.

The challenge of biology in this post genomic era is to elucidate gene function so as to enable identification of potential therapeutic targets or leads.

Recent estimates of the number of individual genes in the human genome (~30,000) and the number of unique putative therapeutic leads attainable using existing chemistries (~100,000 million) suggest over $10^{12}$ assays would be required to completely map the structure-activity of all potential therapeutic targets (1).

*Functional genomics*

A number of screening methods have been developed to enable characterization of protein function. Such methods typically employ various protein-protein interaction assays. Data generated from such assays facilitates elucidation of protein function and as such provides insight into the possible biological roles of previously uncharacterized proteins.

Two large-scale screening methods have proven to be valuable in identifying potential protein-protein interactions, the yeast two-hybrid system and protein mass spectrometry (2).

The yeast two-hybrid system is an artificial transcription-based assay that relies on the principle that many proteins, including transcriptional activators, consist of modular domains that can function independently. When individual domains are expressed separately and then brought into close proximity via non-covalent interactions, such domains can function collectively to reconstitute the activity of the intact protein.

The two-hybrid system can be used to screen libraries of activation domain hybrids to identify proteins that bind to a protein of interest. These screens result in the immediate availability of the cloned gene for any new protein identified. Because multiple clones that encode overlapping regions of proteins are often identified, the minimal domain for interaction may be elucidated from the initial screen (3).

Although the two hybrid method has evolved considerably since first presented (4), it is still mostly limited to proteins that can be localized to the nucleus, thus preventing efficient use with certain extracellular proteins.

In addition, the two hybrid system suffers from several other inherent limitations; first, proteins must be able to fold and exist stably in yeast cells and to retain activity as fusion proteins; second, interactions dependent on post-translational modification that do not occur in yeast, or occur inefficiently, will not be detected;third, many proteins, including those not normally involved in transcription, will activate transcription when fused to a DNA-binding domain and fourth, interactions involving a third non-peptidic factor might not be detected.

While many of the protein-protein interactions are likely to be too weak to be detected by any screening method established to date, recent advances in protein mass-spectrometry have facilitated the identification of protein-protein complexes (5). Proteins and tryptic peptides from these complexes can be analyzed by MALDI-TOF, sequences derived from the mass, and the sequences compared with a database of predicted proteins encoded by the organism's genome. If mass alone cannot predict the exact sequence, fragmentation methods can be used to produce stretches of up to 16 amino acids of sequence (6).

Although a promising approach, the mass spectroscopy method is limited by high costs of operation and equipment and a need for highly skilled technicians. More significantly, this method is also limited by the need for isolated protein complexes, which are oftentimes difficult or nearly impossible to obtain.

Protein function can be also be elucidated by exploring the intracellular localization of a protein. The eukaryotic cell is highly compartmentalized, as are the processes that occur within it, whether involving basic housekeeping activities or more specialized functions.

The presence of numerous organelles, compartments and domains, enables the cell to self-govern the distribution of thousands of molecules in an ordered and precise manner. The regulation of eukaryotic cell function relies on the differential compartmentalization of various cell components. This close relationship between subcellular localization and function enables, at times, to determine protein function on the basis of protein localization.

The identification of proteins that are localized in a given compartment typically requires a lengthy procedure of cell-disruption and ultracentrifugation. Cells can be disrupted by osmotic shock, by ultrasonic vibration, by forcing the cells through a small orifice, or by grinding them up. These procedures disrupt the membranes of the cell but if carefully applied, leave organelles such as nuclei, mitochondria, lysosomes, and peroxisomes intact.

Using such fractionation methods one can isolate subcellular particles, such as organelles, while retaining most of their biochemical properties.

Fractionation approaches suffer from several inherent limitations. First, such approaches depend on the yield and enrichment achievable. Second, purified fractions can be devoid of functionally relevant components lost during purification, while being contaminated with various cell components not normally associated with the fraction. Third, such methodology can only be applied to compartments, which are amenable to cell fractionation, making components exhibiting transient or restricted localization difficult to isolate. Finally, the relative amount and the biochemical characteristics of protein components can impose an additional burden.

### Screening Therapeutic lead compounds

Large-scale screenings for therapeutic leads currently involves performing numerous assays per case. Traditional screening technologies are based on detection of radioactive labels. However radioactive-based screening methods are limited not only by the cost of reagents and as such the cost per assay, but also by the inherent limitations associated with miniaturization of radioactive assays.

Over the years fluorescence and chemiluminescence detection methods have been developed to replace the traditional radioactive detection methods. However, although fluorescence labeling is inherently sensitive, it does not provide adequate performance for large-scale screens since it is susceptible to background effects, both from the biological milieu and from photophysical effects such as light scattering.

Novel fluorescent detection methods have been developed to overcome these difficulties. One extremely versatile and sensitive method that serves broadly as a replacement for radioactivity is based on the time-resolved fluorescence (TRF) measurements of the rare earth Lanthanide ions (LnTRF) such as Europiumropium (Europium). Because the $Eu^{+3}$ label is at least as sensitive as $^{125}$Iodine, which is commonly used in radioactivity assays, this technique has found increasingly broad application, as a replacement for radioactivity in large scale screening. Furthermore, many types of assays that have been developed, using radioactive labels can be switched to Lanthanide-based assays, simply by using different labeling reagents. Examples of radioactive-based assays that have been successfully converted to Lanthanide assays include Europium-labeled streptavidin-based detection of biotinylated targets (7), and tyrosine kinase assays, using Europium-labeled antiphosphotyrosine (8). However LnTRF labeling suffers from several major drawbacks. Assays including such labels are restricted to a pH>7, in order to ensure the integrity of the Europium chelate in the amine-labeling reaction. In addition, this screening method requires the use of an enhancement solution that dissociates Europium from the complex in-order to enhance the Lanthanide fluorescence.

Assays based on FRET (fluorescence resonance energy transfer) from a caged $Eu^{+3}$ to allophycocyanin (APC) further expand the range of the LnTRF method. This method is based on the principle that when two fluorophores with overlapping emission/absorption spectra are within a certain distance of one another and their transition dipoles are appropriately oriented, stimulation of the higher-energy donor fluorophore excites the lower-energy acceptor fluorophore, causing it to emit photons. As implied in the definition, a major consideration in choosing an assay based on energy transfer is the distance change that is induced upon ligand binding or enzyme turnover. For energy transfer to be possible, the distance must be less that about 40-50Å. To put this distance in perspective, 40Å is approximately the diameter of a protein molecule with molecular weight of 26,000 Da. Thus, the sensitivity of FRET to distances on the molecular scale sets a major limitation on its feasibility.

At the cutting edge of large-scale screenings for therapeutic leads is the use of fluorescent cell-based functional methods. Such cytofunctional assays are finding increasing application for large-scale screenings of lead compounds (9).

Functional methods for screening have many advantages over biochemical assays. For example, when screening for receptor binding compounds, functional screens enable the researcher to discriminate between different binding modes (agonist versus antagonist), as well as to broaden the target base to multiple components of a signaling cascade regardless of the degree of biochemical characterization of the pathway.

Although some of the above described methods, of functional characterization and therapeutic lead screening are utilized by both the academic and commercial sectors, there exists a need for novel approaches to characterization of protein function and identification of therapeutic leads which can be easily implemented on a large scale, while being cost effective and accurate.

### SUMMARY OF THE INVENTION

According to one aspect of the present invention there is provided a method of isolating polynucleotides encoding polypeptides affecting an organization of a subcellular organelle or structure of interest, the method comprising: (a) expressing within a plurality of cells an expression library including a plurality of expression constructs each encoding a polypeptide of interest; (b) highlighting the subcellular organelle or structure of interest of the plurality of cells; and (c) isolating a cell or cells of the plurality of cells in which a cellular distribution and/or level of the subcellular organelle or structure of interest is altered to thereby isolate polypeptides capable of affecting the organization of the subcellular organelle or structure of interest.

According to another aspect of the present invention there is provided a method of isolating polynucleotides encoding polypeptides capable of localizing to a subcellular organelle or structure of interest, the method comprising: (a) expressing within a plurality of cells an expression library including a plurality of expression constructs each encoding a polypeptide of interest fused to a polypeptide label; (b) localizing the polypeptide of interest within the plurality of cells via the polypeptide label; and (c) isolating a cell or cells of the plurality of cells in which the polypeptide of interest is localized to the subcellular organelle or structure of interest, to thereby isolate polynucleotides encoding polypeptides capable of localizing to the subcellular organelle or structure of interest.

According to yet another aspect of the present invention there is provided a method of isolating polynucleotides encoding polypeptides being localized to a subcellular organelle or structure of interest, the method comprising: (a) expressing within a plurality of cells an expression library including a plurality of expression constructs each encoding a polypeptide of interest fused to a polypeptide label; (b) highlighting the subcellular organelle or structure of interest of the plurality of cells; (c) localizing the polypeptide of interest within the plurality of cells via the polypeptide label; and (d) isolating a cell or cells of the plurality of cells in which the polypeptide of interest is colocalized with the highlighted subcellular organelle or structure of interest to thereby isolate polynucleotides encoding polypeptides being localized to the subcellular organelle or structure of interest.

According to still another aspect of the present invention there is provided a method of isolating polynucleotides encoding polypeptides which specifically bind a target polypeptide, the method comprising: (a) expressing within a plurality of cells an expression library including a plurality of expression constructs each encoding a polypeptide of interest fused to a first polypeptide label; (b) providing within the plurality of cells a chimeric polypeptide including: (i) a second polypeptide label being distinguishable from the first polypeptide label; (ii) a polypeptide domain for localizing the chimeric polypeptide to a specific subcellular organelle or structure; and (iii) at least a portion of the target polypeptide; and (c) localizing the polypeptide of interest and the chimeric polypeptide within the plurality of cells via each of the first and second polypeptide labels; and (d) isolating a cell or cells of the plurality of cells in which the polypeptide of interest colocalizes with the chimeric polypeptide, thereby isolating polynucleotides encoding polypeptides which specifically bind the target polypeptide.

According to further features in preferred embodiments of the invention described below the step of highlighting is effected by at least one method selected from the group consisting of: (i) labeling an endogenous molecule associated with, or forming a part of, the subcellular organelle or structure of interest; and (ii) providing within the plurality of cells a labeled molecule capable of associating with the subcellular organelle or structure of interest.

According to still further features in the described preferred embodiments a label of the labeled molecule is selected from the group consisting of a fluorescent label, a radioactive label, an epitope label, a biotin label and an enzyme.

According to still further features in the described preferred embodiments the labeled molecule is a labeled polypeptide and further wherein providing within the plurality of cells the reporter polypeptide is effected by expressing within the plurality of cells a nucleic acid construct for encoding the labeled polypeptide.

According to still further features in the described preferred embodiments the endogenous molecule is selected from the group consisting of a protein, a lipid, a second messenger, an ion, a free radical, a subcellular organelle and a structure.

According to still further features in the described preferred embodiments the polypeptide label is selected from the group consisting of an epitope tag, a fluorescent protein and an enzyme.

According to still further features in the described preferred embodiments the cell is a mammalian cell line.

According to still further features in the described preferred embodiments the method further comprising a step of fixing the plurality of cells prior to step (b).

According to still further features in the described preferred embodiments the step of isolating the cell or cells of the plurality of cells in which the polypeptide label is colocalized with the highlighted subcellular organelle or structure of interest is effected by digital microscopy combined with cross correlation image processing.

According to still further features in the described preferred embodiments each of the first and second polypeptide labels is independently selected from the group consisting of an epitope tag, a fluorescent protein and an enzyme.

According to still further features in the described preferred embodiments the step of providing within the plurality of cells the chimeric polypeptide is effected by introducing into the plurality of cells an expression construct expressing the chimeric polypeptide.

According to an additional aspect of the present invention there is provided a nucleic acid construct comprising a polynucleotide region encoding a chimeric polypeptide including: (a) a polypeptide label; (b) a polypeptide domain for localizing the chimeric polypeptide to a specific subcellular organelle or structure; and (c) at least a portion of a polypeptide of interest.

According to yet an additional aspect of the present invention there is provided a reporter cell comprising an expression construct encoding a chimeric polypeptide including: (a) a polypeptide label; (b) a polypeptide domain for localizing the chimeric polypeptide to a specific subcellular organelle or structure; and (c) at least portion of a polypeptide of interest.

According to still an additional aspect of the present invention there is provided a method of identifying at least one agent capable of at least partially reversing an abnormal cellular phenotype, the method comprising: (a) exposing the cell characterized by the abnormal phenotype to the at least one agent; and (b) monitoring a change in at least one cellular constituent being associated with the abnormal cellular phenotype, the change being indicative of at least a partial phenotype reversal, to thereby determine the capability of the at least one agent in at least partially reversing the abnormal cellular phenotype.

According to still further features in the described preferred embodiments the method further comprising a step a highlighting the at least one cellular constituent, wherein the step of highlighting is effected by at least one method selected from the group consisting of: (i) labeling the at least one cellular constituent being associated with the abnormal cellular phenotype; (ii) providing the at least one cellular constituent being associated with the abnormal cellular phenotype within the cell, wherein the at least one cellular constituent being fused to a label.

According to still further features in the described preferred embodiments the providing the at least one cellular constituent is effected by introducing into the cell the at least one cellular constituent or a nucleic acid construct for expressing the at least one cellular constituent.

According to still further features in the described preferred embodiments the change in the at least one cellular constituent includes a change in an at least one parameter selected from the group consisting of a cellular distribution, a biochemical modification, an expression level and an activity of the at least one cellular constituent.

According to still further features in the described preferred embodiments the step of monitoring a change in the at least one cellular constituent is effected by at least one method selected from the group consisting of: (i) comparing the at least one cellular constituent of the cell with that of a second cell, characterized by a normal cell phenotype; (ii) comparing the at least one cellular constituent within the cell prior to step (a), and following step (b).

According to still further features in the described preferred embodiments the at least one agent is selected from the group consisting of a test condition and a test compound.

According to still further features in the described preferred embodiments the test condition is selected from the group consisting of a growth condition and a radiation condition.

According to still further features in the described preferred embodiments the test compound is selected from the group consisting of a synthetic product and a natural product.

According to still further features in the described preferred embodiments the at least one cellular constituent is selected from the group consisting of a protein, a lipid, a second messenger, an ion, a free radical, a subcellular organelle and a structure.

According to still further features in the described preferred embodiments the method further comprising a step of generating the cell characterized by the abnormal cellular phenotype, prior to step (a).

According to still further features in the described preferred embodiments the cell characterized by the abnormal cellular phenotype is of a pathological origin.

According to a further aspect of the present invention there is provided a method of quantifying a co-localization of a plurality of distinguishable molecules in a cell, the method comprising the steps of: (a) acquiring an image of the cell for each of the plurality of distinguishable molecules so as to obtain a plurality of images of the cell, each individual image of the plurality of images presenting a distribution of one of the plurality of distinguishable molecules in the cell; and (b) calculating a correlation coefficient for at least one pair of the individual images, thereby quantifying the co-localization of at least a pair of the distinguishable molecules in the cell.

According to yet a further aspect of the present invention there is provided a method of monitoring a localization of a molecules in a cell, the method comprising the steps of: (a) acquiring a plurality of images of the cell at different time points, each of the plurality of images presenting a distribution of the molecule in the cell; and (b) calculating a correlation coefficient for at least one pair of images of the plurality of images, thereby monitoring the localization of the molecule in the cell.

According to still further features in the described preferred embodiments each of the plurality of images is a normalized image.

According to still further features in the described preferred embodiments the method comprising the steps of: (c) prior to step (b), calculating a plurality of distortion vectors, one for respective subregions of each pair of the images, the distortion vectors being selected so as to maximize regional correlation coefficients of each of the pair of respective subregions; and (d) using the plurality distortion vectors to correct for distortions in the at least one pair of images of the plurality of images.

According to still a further aspect of the present invention there is provided a system for quantifying a co-localization of a plurality of distinguishable molecules in a cell, the system comprising a data processor for: (a) acquiring an image of the cell for each of the plurality of distinguishable molecules so as to obtain a plurality of images of the cell, each individual image of the plurality of images presenting a distribution of one of the plurality of distinguishable molecules in the cell; and (b) calculating a correlation coefficient for at least one pair of the individual images, thereby quantifying the co-localization of at least a pair of the distinguishable molecules in the cell.

According to yet a further aspect of the present invention there is provided a system for monitoring a localization of a molecule in a cell, the system comprising a data processor for: (a) acquiring a plurality of images of the cell at different time points, each of the plurality of images presenting a distribution of the molecule in the cell; and (b) calculating a correlation coefficient for at least one pair of images of the plurality of images, thereby monitoring the localization of the molecule in the cell.

According to further features in preferred embodiments of the invention described below the data processor is further for: (c) calculating a plurality distortion vectors, one for respective subregions of each pair of the images, the distortion vectors being selected so as to maximize regional correlation coefficients of each of the pair of respective subregions; and (d) using the plurality distortion vectors to correct for distortions in the at least one pair of images of the plurality of images.

The present invention successfully addresses the shortcomings of the presently known configurations by providing novel screening methods, which enable on the one hand, to elucidate new cellular targets, based on their subcellular localization, and on the other hand to discover new therapeutic leads. The methods of the present invention are readily applicable for high-throughput screening assays based on robotic preparations of samples in multi-wells and their automated imaging and real-time analysis by computerized microscopy. Combination of these methods and adaptation of present capacities in microscope technologies to fast screening of microsamples are estimated to test in exceess of 100,000 samples per day. This allows to obtain fast feedback from screens of gene and drug libraries.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

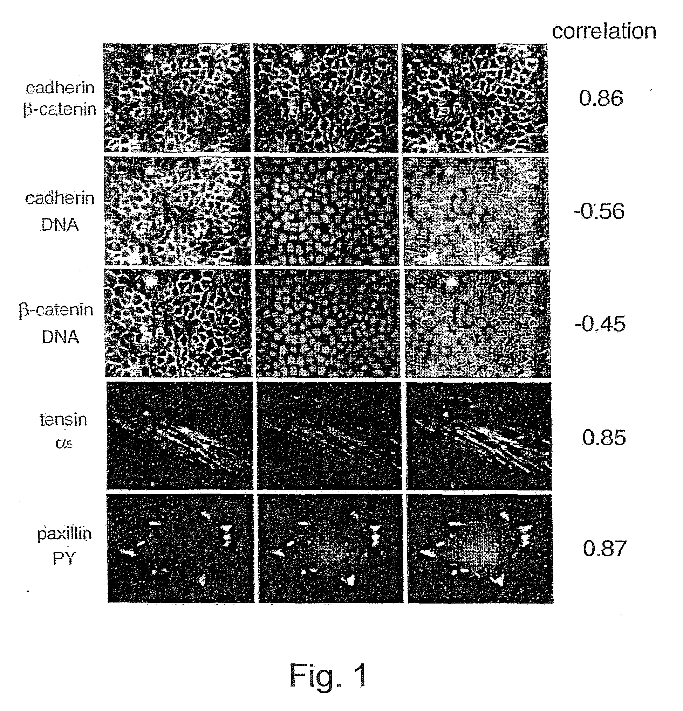

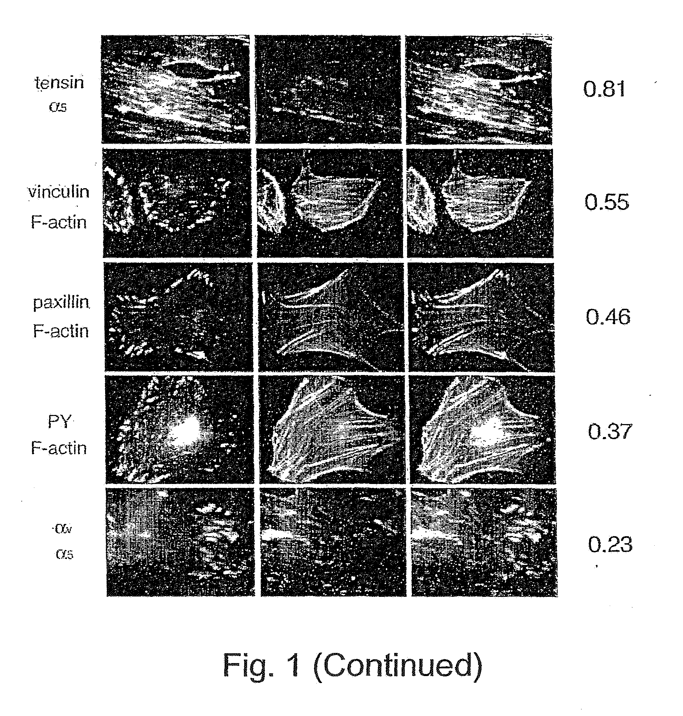

FIG. 1 demonstrates correlation analysis to score the co-localization degree of two different cellular components. Cells were fixed and double-labeled for two different components, as indicated for each row, using fluorophores with different colors: red (Cy3), green (FITC) or blue (DAPI). Images of the labeling of each component in the double-labeled cells were acquired and are presented in the first and second columns. The third column shows the super-position of the two labeling. The names of the components, as well as their images, are colored according to their labeling color. In each row, the correlation between the left image and the middle image was calculated (according to Eq. 1, where M and N are the two images) and are presented.

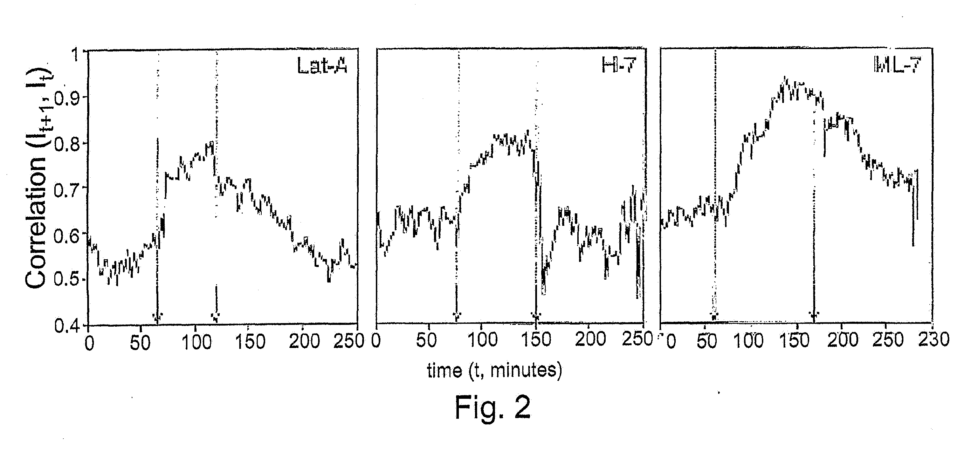

FIG. 2 shows a correlation analysis of the affect of acto-myosin inhibitors on fibrillar adhesion dynamics- HFF cells were analyzed by time-lapse fluorescence recording starting 24 hours after transfection with GFP-tensin as described (10). During the recording different inhibitors of acto-myosin contractility were added (2 mM Lat-A, 150 mM H-7 or 100 mM ML-7) at the time point indicated by the first arrow, and washed out at the time point indicated by the second arrow. In each digital movie, a constant square frame (I) of $285\mu$ m$^2$ containing only fibrillar adhesions without focal contacts was acquired as a function of time. Correlation was calculated between pairs of subsequent time points (It and $I_{t+1}$) for the duration of the movie. Correlation between It and $I_{t+1}$ was calculated (according to Eq. 1, where N=It and M=$I_{t+1}$). The graphs present this correlation as a function of time (t).

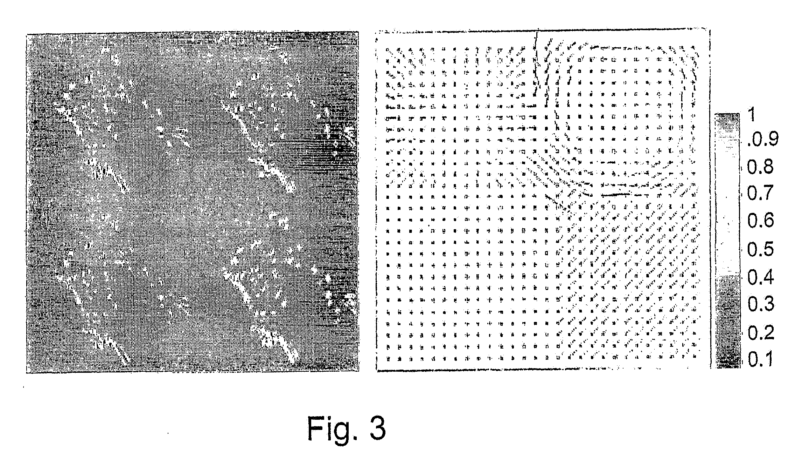

FIG. 3 shows correlation analysis of simulated translation, contraction or rotation distortions. A digital image of a single HFF cell, stained for paxillin, was acquired. The original image was then digitally processed to obtain three simulated distortions: either rotated, contracted or translated image. Two composite images were made: one composed from 4 copies of the original image (green) and the second is composed from the rotated, contracted, translated and original images (red). The image on the left shows the super-position of these two composite images. The images were divided to 784 partially overlapping sub-image square frames. For each frame, the translation vector, ($[a^{max}, b^{max}]$, Eq. 4), that maximizes the correlation (C, Eq. 2, where N and M correspond to the same frame in the two composites) between the green and the red images was found. The translation vector, ($a^{max}, b^{max}$), of each frame is presented on the right figure by a line starting at the center of the frame with length and direction equal to the vector. The color of the line presents the value of the maximal correlation found, $C^{max}$, encoded by a color spectrum scale.

DESCRIPTION OF THE PREFERRED EMBODIMENTS

The present invention is of methods for isolating genes encoding proteins of specific function. Specifically, the present invention can be used to isolate genes encoding proteins which are localized to specific subcellular organelles or structures, proteins involved in the formation, organization, modulation and/or maintenance of specific subcellular organelles or structures and proteins which bind other proteins.

The principles and operation of methods according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

The ability to resolve a function of a protein encoded from a given DNA sequence is a fundamental goal in biotechnfllogy. Current techniques are essentially based on biochemical protocols, elucidating protein function from either protein-protein interaction maps or protein localization. Such biochemical techniques are laborious, time consuming and often result in false interpretations.

The present invention provides a novel approach for elucidating protein function. As described hereinunder and in the Examples section which follows, the present invention provides novel visual screening methods which can be used for identifying the effect of a protein of interest on cellular phenotype even in cases where such an effect is minimal As used herein the term "polypeptide" refers to an amino acid polymer of a length anywhere between a few or several amino acids to several thousand amino acids, which can represent either a fraction or an entire sequence of a characterized or uncharacterized protein from any source or organism. It will be appreciated that although polynucleotide fragments originating either from genomic or mRNA sources are preferably utilized to code for the polypeptides employed by the present invention, combinatorial polynucleotide sequences, which, for example, can be synthetic or shuffled DNA segments of different lengths can also be employed. Thus, the term polypeptide is also used herein to refer to chimeras and combinatorial polypeptides encoded by polynucleotides produced by various synthesis methods, which are well known in the art.

As used herein the phrase "label molecule" refers to a molecule, which exhibits a quantifiable activity or characteristic. A label molecule may be a "polypeptide label" such as a polypeptide, which, can be quantitated either directly or indirectly. For example, a polypeptide label can be an enzyme which when in the presence of a suitable substrate generates chromogenic products, Such enzymes include but are not limited to alkaline phosphatase, β-galactosidase, β-D-glucoronidase (GUS) and the like. A polypeptide label can also be a fluorescer such as the polypeptides belonging to the green fluorescent protein family including the green fluorescent protein, the yellow fluorescent protein, the cyan fluorescent protein and the red fluorescent protein as well as their enhanced derivatives. In such case, the polypeptide label can be quantified via its fluorescence, which is generated upon the application of a suitable excitatory light. Alternatively, a polypeptide label can be an epitope tag, a fairly unique polypeptide sequence to which a specific antibody can bind without substantially cross reacting with other cellular epitopes. Such epitope tags include a Myc tag, a Flag tag, a His tag, a Leucine tag, an IgG tag, a streptavidin tag and the like. Further detail of polypeptide labels can be found in Misawa et al. (11).

Alternatively a labeled molecule can be a chemical, which may be detected directly or indirectly such as radioisotopes, or biotin molecules.

A labeled molecule can be also a dye. A diverse array of cell permeant fluorescent dyes as well as perfused cell dyes, are capable of selectively associating with cellular constituents in living cells. Examples include but are not limited to, subcellular organelles and structures stains, lipid stains such as fluorescent analogs for natural lipids (e.g., phospholipids, sphingolipids, fatty acids, triglycerides and steroids), probes for detecting various reactive oxygen species (such as hydroperoxides in living cells membranes) and fluorescent indicators for ion detection such as, magnesium, sodium, potassium, hydrogen, zinc, chloride protons etc. Such dyes are well known in the art and are commercially available from for example, molecular probes, [for example see, "Handbook of Fluorescent Probes and Research Chemicals" (www.molecularprobes.com/handbooklsections/1200.html), Chapter 11 — Probes for Actin, Tubulin and Nucleotide-Binding Proteins, Chapter 12 — Probes for Organelles, Chapter 13 — Probes for Lipids and Membranes, Chapter 18 — Probes for Signal Transduction, Chapter 19 — Probes for Reactive Oxygen Species, Including Nitric Oxide, Chapter 20 — Indicators for $Ca^{2+}$, $Mg^{2+}$, $Zn^{2+}$ and Other Metals, Chapter 21 — pH Indicators].

As used herein, the phrase "cis acting regulatory element" refers to a polynucleotide sequence, which binds a trans acting regulator and regulates the transcription of a coding sequence located down stream thereto. For example, a transcriptional regulatory element can be a part of a promoter sequence which is activated by a specific transcriptional regulator or it can be an enhancer which can be adjacent or distant to a promoter sequence and which function in up regulating the transcription therefrom. A cis acting regulatory element can also be a translational regulatory sequence element in which case such a sequence can bind a translational regulator, which up regulates translation.

The term "expression" refers to the biosynthesis of a gene product. For example, in the case of a structural gene, expression involves the transcription of the structural gene into messenger RNA (mRNA) and the translation of the mRNA into one or more polypeptides

According to one aspect of the present invention there is provided a method of isolating polynucleotides encoding polypeptides, which are localized to a subcellular organelle or structure of interest. Such subcellular organelle or structure of interest can, for example, be a cell membrane, a cell nucleus, cell chromatin, mitochondria, chloroplastids, endoplasmic reticulum, golgi apparatus, lysosomes, secretion vesicles, focal adhesion structures, adherens junction structures, tight junctions, desmosomes, intermediate filaments, microfilament structures or microtubule structures.

The method according to this aspect of the present invention is effected by several steps. In a first step an expression library is prepared.

The expression library includes a plurality of expression constructs. Each of the expression constructs of the expression library includes a first polynucleotide, which encodes a polypeptide of a plurality of polypeptides, which, are tested for localization to the subcellular organelle, or structure of interest. The first polynucleotides can be cDNA fragments obtained by reverse transcribing and optionally PCR amplifying mRNA isolated from any one or more cells, tissues or organisms, it can be a synthetic nucleic acid, or it can be a fragmented nucleic acid derived from a genome. The first polynucleotide can be relatively short, encoding for several amino acids, or longer, encoding for tens, hundreds or thousands of amino acids. There is no particular limitation for the length of the first polynucleotide. In one example, the source for mRNA is foreskin fibroblasts (primary), human Umbilical Cord Vein Endothelial cells (HUVEC) and/or human epithelial cells (HeLa or MCF-7).

Each of the expression constructs of the expression library according to this aspect of the invention further includes an in-framed second polynucleotide ligated upstream or downstream to the first polynucleotide and which encodes a polypeptide label, described hereinabove. Though the polypeptide label is of no significance, it will be appreciated that the label should not alter the three dimensional structure of the polypeptides tested for subcellular localization.

Each of the expression constructs of the expression library according to this aspect of the invention further includes at least one cis acting regulatory element, e.g., a promoter and an enhancer, for directing expression of a chimeric protein from the construct. The promoter of choice that is used in conjunction with this invention is of secondary importance, and will comprise any suitable promoter. It will be appreciated by one skilled in the art, however, that it is necessary to make sure that the transcription start site(s) will be located upstream of an open reading frame. In a preferred embodiment of the present invention, the promoter that is selected comprises an element that is active in the particular host cells of interest. These elements may be selected from transcriptional regulators that activate the transcription of genes essential for the survival of these cells in conditions of stress or starvation, including the heat shock proteins.

A construct according to the present invention preferably further includes an appropriate selectable marker. In a more preferred embodiment according to the present invention the construct further includes an origin of replication. In another most preferred embodiment according to the present invention the construct is a shuttle vector, which can propagate both in E. coli (wherein the construct comprises an appropriate selectable marker and origin of replication) and be compatible for propagation in cells, or integration in the genome, of an organism of choice. The construct according to this aspect of the present invention can be, for example, a plasmid, a bacmid, a phagemid, a cosmid, a phage, a virus or an artificial chromosome.

It is well known that in the course of preparation of a library as herein described many individual constructs may be inoperative due to, for example, out of frame ligations, etc. This however, can be readily overcome by increasing library size, thereby enabling sufficient representation of operative constructs.

Thus, the expression library according to this aspect of the present invention encodes a variety of chimeric proteins, each including a unique sequence fused to a polypeptide label. In the next step of the method according to this aspect of the present invention the expression library is introduced into a plurality of cells. Measures are taken to control the number of constructs entering a particular cell, preferably a single construct is introduced into each individual cell.. A wide variety of cell types can be employed by the present invention . Examples include cells such as fibroblasts, epithelial cells, endothelial cells, lymphoid cells, neuronal cells and the like. Such cells should be readily propagatable in culture. Specific examples thus include, but are not limited to, various cell lines such as 293T, NIH3T3, H5V, CHO, HeLa and L-cells, etc.

Following an incubation time sufficient for protein de novo-synthesis, the localization of the chimeric polypeptide in the transfected (infected) or transformed cells is determined via the polypeptide label. According to one embodiment of this aspect of the present invention this may be effected directly by using an intrinsically fluorescent label.

According to another embodiment of this aspect of the present invention localizing the polypeptide label requires contacting the transformed cells with a fluorogenic or chromogenic label (i.e., a dye or a fluorescent antibody), which is capable of generating a detectable signal. This oftentimes requires an additional step of permeating the transformed cells prior to staining. Cell permeabilizing fixing protocols are well known in the art and are specified for example in (12).

Thereafter, a screening procedure (manual or automatic) is used to isolate a cell or cells in which the polypeptide label is localized to the subcellular organelle or structure of interest. Isolated cells are propagated and the polynucleotides encoding the polypeptides localized to the subcellular organelle or structure of interest are isolated therefrom using methods, such as, PCR amplification, so as to obtain an isolated polynucleotides encoding such proteins.

PCR amplification can be readily effected since the sequences flanking the polynucleotide to be isolated are known and suitable amplification primers can therefore be designed. PCR amplification protocols can be found in, for example, PCR Protocols: A Guide To Methods And Applications", Academic Press, San Diego, CA (1990).

Screening for cell or cells in which the polypeptide label is localized to the subcellular organelle or structure of interest can be effected manually using, for example, a microscope. Alternatively, automatic high throughput screening can also be effected using a microscope combined with a digital camera and any one of a number of pattern recognition algorithms, such as the product distributed under the commercial name ARAYSCAN by Cellomics Inc., U.S.A

Thus, in one example, infected cells are distributed into flat glass-bottom multiwell (96) plates at a precalibrated density that allows the growth of just one or two clones per well. In a typical experiment, between 10-100 plates are prepared and examined microscopically. This screen can be carried out manually. However, the possibility of installing an automated stage, for example multiwell attachment for the Delta Vision microscope, Cellomics automated microscope, or an equivalent.

Furthermore, examination of the cells (not just fluorescent protein distribution) may point to specific changes, detectable by, for example, phase contrast microscopy (e.g., apoptosis, loss of junctions, elongation or rounding up, etc.).

Once identified, cells in positive wells are recloned into multiwell plates and are also plated on glass coverslips for detailed immunocytochemical study.

Cells that are of interest for further study are stored frozen, or extensively characterized by double labeling with relevant antibodies, for example, a variety of cytoskeletal and juncional labeling antibodies.

cDNA from selected clones is retrieved as described above. Amplified cDNA is recloned into retroviral vectors and used for another round of selection and results verification.

cDNA obtained from clones with established phenotypes is retrieved and sequenced. When new proteins/ genes are discovered, the insert DNA is used either for cDNA library screening, or RACE (rapid amplification of cDNA ends) to obtain the full-length cDNA.

Although whole cell-based screening methods aimed at unveiling gene product function are known in the art (WO 99/24563, WO 00/61809, WO 00/39346, WO 99/53098), such methods oftentimes make use of very general phenotypes as a screening approach (i.e., such as cell-growth, death, differentiation, survival, transformation and the like). Such prior art methods cannot be used to detect intermediate or null phenotypes and as such, cannot be used to uncover gene products which give rise to such phenotypes.

In sharp contrast, the visual screening method of the present invention, utilizes a screening output which is in the level of sub-cellular organization or architecture, and as such can yield much higher screening sensitivity due to the following: First, effects can be detected much earlier, as compared to cell-death, survival or transformation. Second, such a visual output can be more specific and involve a narrow range of cellular mechanisms. Finally, even subtle or transient changes can be detected.

The visual screening method of the present invention will lead to the discovery of novel genes encoding novel proteins associated with specific subcellular organelles or structures, such as cytoskeletal and adhesion-associated protein. In principle, the use of cDNAs from a variety of cellular sources, and given that the fusion proteins retain the binding capacity of the native protein, the proposed assay may reveal all, or most, of the proteins associated with the particular sub-cellular site. Such discoveries may provide a unique opportunity to better understand the molecular basis for the assembly and function of such sites and the roles of the particular proteins.

An improvement of the above method constitutes another aspect of the present invention. Accordingly there is provided an improved method of isolating polynucleotides encoding polypeptides which are localized to a subcellular organelle or structure of interest. The method according to this aspect of the present invention is effected by implementing the following method steps. In a first step an expression library is prepared which is similar to the expression library described above with respect to the first aspect of the invention.

In a second step of the method according to this aspect of the present invention the expression library is introduced into a plurality of cells. The difference between this aspect of the invention and the former aspect of the invention is that in this aspect screening is effected using two labels, where one label is the polypeptide label identifying the unknown polypeptides encoded from the library constructs, while the second label identify the subcellular organelle or structure of interest. Since it important to be able to separately localize each of the labels in the cell, the two labels are selected so as to be distinguishable.

In a preferred embodiment of the present invention labeling the subcellular organelle or structure of interest is effected using a specific dye or labeled antibody.

In another preferred embodiment, labeling the subcellular organelle or structure of interest is effected using a chimeric polypeptide, which includes a second polypeptide label and a fusion polypeptide for localizing the chimeric polypeptide to the subcellular organelle or structure of interest. Such expression and localization of the chimeric polypeptide can be achieved by transforming the cells with an expression construct encoding the second chimeric polypeptide. Alternatively the chimeric polypeptide can be introduced into the cell as a polypeptide, via for example, liposome delivery (13), peptide microinjection (14), micropricking (15) or ionophoresis (16). In such cases, the polypeptide can be expressed and collected from another cell system as a native polypeptide, or it can be synthesized in-vitro via well known prior art methods. The synthetic peptide can include modifications rendering the polypeptide more stable while in the cell.

Table 1 below provides some examples for fused polypeptides which can be employed to localize the chimeric polypeptide to particular subcellular organelles or structures:

*Table I*

| Nucleus | histones, lamins |
|---|---|
| Mitochondria | cytochrome oxidase |
| Focal adhesions | vinculin, paxillin |
| Adherens junctions | $\alpha$-catenin |
| Microfilaments | actin, $\alpha$-actinin |
| Microtubulys | tubulin |
| Lysosomes | cathepsin-D |

Following labeling, a magnifying optical device, typically equipped with distinguishing filters for the different polypeptide labels or dyes, is used for screening for a cell or cells of the plurality of cells in which the first polypeptide label is colocalized with the second polypeptide label or alternatively with the dye. Cells in which the first polypeptide label is colocalized with the second polypeptide label or with the dye are used for recovering the first polynucleotide, via, for example, PCR, thereby isolating the polynucleotides encoding the proteins localized to the subcellular organelle or structure of interest.

According to a preferred embodiment of this aspect of the present invention, the step of screening for the cell or cells of the plurality of cells in which the first polypeptide label is colocalized with the second polypeptide label or the dye is effected by digital microscopy combined with cross correlation image processing software as is further detailed hereinunder.

According to yet another aspect of the present invention there is provided a method of isolating polynucleotides encoding polypeptides affecting the organization of a subcellular organelle or structure of interest. The method according to this aspect of the present invention is effected by implementing the following method steps, in which, in a first step an expression library is prepared including a plurality of expression constructs each encoding one of a plurality of polypeptides which is tested for affecting the organization of the subcellular organelle or structure of interest each placed under at least one cis acting regulatory element for directing expression of the polypeptides from the constructs.

In the next step the expression library is introduced into a plurality of cells in which the subcellular organelle or structure of interest is labeled, as described hereinabove.

Finally, a magnifying optical device is used to screen for a cell or cells of the plurality of cells in which a cellular distribution or amount of the polypeptide label or the in vivo dye is affected. Such cells are isolated, propagated, and the polynucleotides encoding the proteins affecting the organization of the subcellular organelle or structure of interest are isolated as described above.

Screening for the cell or cells in which the cellular distribution or amount of the polypeptide label or dye is affected can be performed manually using a microscope. However, since the staining patterns of specific cellular organelles and structures are well known, using a microscope combined with a digital camera and any one of a number of pattern recognition algorithms can be used to identify cells which have a different distribution patterns of the polypeptide label or in vivo dye.

According to still another aspect of the present invention there is provided a method of isolating polynucleotides encoding polypeptides which specifically bind a target protein of interest. The method according to this aspect of the present invention is effected by implementing the following method steps, in which in a first step an expression library is prepared, including a plurality of expression constructs each having (i) a first polynucleotide encoding a polypeptide of a plurality of polypeptides being tested for specific binding to the target protein of interest; (ii) a second polynucleotide encoding a first polypeptide label, the second polynucleotide being ligated upstream or downstream to the first polynucleotide; and (iii) at least one cis acting regulatory element for directing expression of a first chimeric protein from the construct.

Then, the expression library is introduced into a plurality of cells expressing a second chimeric protein which includes (i) a second polypeptide label which is distinguishable from the first polypeptide label; (ii) a fused polypeptide for localizing, e.g., anchoring, the second chimeric protein to a predefined subcellular organelle or structure; and (iii) at least a fused portion of the target protein.

Each of the chimeric polypeptides is localized within the cell via each of the polypeptide labels, thereafter, a magnifying optical device is used for screening for a cell or cells of the plurality of cells in which the first polypeptide label is colocalized with the second polypeptide label. Cells in which the first polypeptide label is colocalized with the second polypeptide label are likely to be cells in which the tested polypeptide specifically binds the target protein. Such cells are thereafter propagated and the polynucleotides encoding the polypeptides which, specifically bind the target protein are isolated. Specific binding of the polypeptides encoded by the isolated polynucleotides to the target protein may thereafter be substantiated using methods well known in the art, such as, but not limited to, gel retardation, co-immunopercipitation, affinity columns and the like. If no binding is detected, then the isolated polynucleotide encodes a protein, which is colocalized to the predefined subcellular organelle or structure, but do not bind directly to the target protein in under the test conditions, or alternatively binds another protein that binds the target polypeptide.

In accordance with this aspect of the present invention, there is also provided an expression construct for expressing a chimeric protein, and a reporter cell comprising such an expression construct. The expression construct including (a) a first polynucleotide encoding a polypeptide label; (b) an in frame second polynucleotide encoding a polypeptide for localizing the chimeric protein to a predefined subcellular organelle or structure; and (c) an in frame third polynucleotide encoding at least a fused portion of a target protein; and a reporter cell system expressing same.

According to a preferred embodiment of this aspect of the present invention, the step of screening for the cell or cells of the plurality of cells in which the first polypeptide label is colocalized with the second polypeptide label is effected by digital microscopy combined with cross correlation image processing as is further detailed herein under.

The following is a description of a novel approach for quantification of co-localization of molecules in live or fixed cells which novel approach can be used to implement the aspects of the present invention in which two distinctive polypeptide labels are employed and colocalization thereof to a specific subcellular organelle or structure is indicative of positive and interesting results. Thus, this novel approach enables automatic identification of subcellular localization sites of molecules. When examining live cells, it enables determination of changes in the location of specific molecules in the cells over time.

For comparison of two components, cells can be either transfected with two fluorescent proteins and examined live or fixed, or fixed first and then double immunolabeled or stained. The two fluorescent images will then be acquired using high-resolution CCD camera and suitable filters and the two images processed (see below). A correlation analysis between the images determines a global correlation coefficient, which is indicative of the level of co-localization. In principle, a correlation value of 1 indicates identity between the two images, -1 indicates inverse distribution ("positive-negative relationships") and "0" indicates no correlation.

For studying dynamic changes, images of live cells, expressing one or more fluorescent components, are acquired at different time points and compared as above.

For detecting, measuring and correcting translational and rotational shifts and other distortions between similar images showing the same field at different time points or with different labeling, translation vectors which maximize local correlation in sub-regions between the two frames are calculated and used. The collection of these translation vectors is indicative of the distortion between the images and provides the mathematical means to correct it.

The basic idea of this novel approach is that correlation is a quantitative measure of image similarity. Correlation is defined as the normalized pixel-by-pixel multiplication of two normalized images M and N (Eq. 1), and it is higher when the similarity between the two images is higher. Thus, if, and only if, N and M are identical images the correlation gets its maximum value of I. When N equals -M (inverted images) the correlation reaches its minimum value, -1, and when N and M are totally unrelated images the correlation approaches zero. The below correlation equation (Eq. 1) can be extended to evaluate the similarity of two images which, are displaced with respect to each other (Eq. 2). To illustrate how this can be applied to characterize dynamic processes consider 4 cases in which N and M are two images of the same field taken at two close time points: (i) a correlation which equals 1 indicates a lack of any change in the field (since N equals M); (ii) if all the objects in the field are translated one pixel along the x axis, such that $M_{x,y}=N_{x+1,y}$, the correlation defined above will be less then 1. Yet when the objects are bigger then one pixel they will overlap in the two images, and thus the value of the correlation between N and M will be still close to 1; (iii) if the objects are translated 100 pixels, such that $M_{x,y}=N_{x+100,y}$ with no overlap between the objects in the two images, the correlation will be low. However there is a single translational vector ($[a^{max},b^{max}]$, Eq. 4), i.e. (1,0) for case (ii) and (100,0) for case (iii), that will bring the two images to perfectly overlap, $M_{x,y}=N_{x+a,y+b}$, and will raise the more general translated correlation (C, Eq. 2) back to its maximal value of unity; (iv) if the objects moved 100 pixels, but different ones moved to different directions, the correlation will be low for all possible translational vectors, since the two images never overlap.

In this way, the speed of objects translocation can be scored by a simple correlation (Eq. 1). If the translocation is uniform in speed and direction (as will be implied by $C^{max}$ close to 1, Eq. 5) then tracking it is possible by the translation vector [$a^{max}$, $b^{max}$] (Eq. 4). On the other hand, a low $C^{max}$ will indicate a variable translocation within the frame.

$$correlation = \frac{\sum_{x=-w}^{w}\sum_{y=-l}^{l}\left((M_{x,y}-\overline{M})*(N_{x,y}-\overline{N})\right)}{\sqrt{\left(\sum_{x=-w}^{w}\sum_{y=-l}^{l}(M_{x,y}-\overline{M})^2\right)*\left(\sum_{x=-w}^{w}\sum_{y=-l}^{l}(N_{x,y}-\overline{N})^2\right)}} \quad \text{Eq. 1}$$

$$C_{a,b} = \frac{\sum_{x=-w}^{w}\sum_{y=-l}^{l}\left((M_{x,y}-\overline{M})*(N_{x+a,y+b}-\overline{N})\right)}{\sqrt{\left(\sum_{x=-w}^{w}\sum_{y=-l}^{l}(M_{x,y}-\overline{M})^2\right)*\left(\sum_{x=-w}^{w}\sum_{y=-l}^{l}(N_{x+a,y+b}-\overline{N})^2\right)}} \quad \text{Eq. 2}$$

$$\left\{(a,b)\in Z^2 \mid (-H \leq a \leq H),(-H \leq b \leq H)\right\}=D \quad \text{Eq. 3}$$

$$(a^{max},b^{max}) = \left\{(i,k)\in D \mid C_{i,k} = \max(C_{f,g})\forall(f,g)\in D\right\} \quad \text{Eq. 4}$$

$$C^{max} = C_{a^{max},b^{max}} \quad \text{Eq. 5}$$

M and N are two (2W+1)*(2L+1) rectangle images. $M_{x,y}$ and $N_{x,y}$ are the intensities of pixel (x,y) in these images. $C_{a,b}$ is the normalized correlation between M and translated N along (x,y) by the translation vector (a,b). H limits the searching range for maximizing $C_{a,b}$, and D is the resulted group of allowed (a,b) vectors. The maximal correlation score found is $C^{max}$ and the corresponding translation vector is ($a^{max}$,$b^{max}$). A highly sensitive and refined version of the correlation is introduced, where the sums (x,y) are limited to the regions in the images where wither $M_{x,y}$ or $N_{x,y}$ have intensities higher than the background.

The methods of the present invention described above provide methods of isolating genes whose protein products have specific intracellular localization. This visual screening method is of particular significance, since it benefits from the close relationship between subcellular localization and function. Thus, determining the preferential localization of a gene product is an essential step towards understanding its function. Moreover, the DNA sequences encoding proteins of particular localization pattern can be directly cloned from these cells. This visual cell-based screening method may be also used for isolation of agents of therapeutic potential. A screening method based on imaging techniques, tracking cellular phenotype, enables a direct correlation between potential therapeutic leads identified in the screen and possible diseases and syndromes which may be treated with such, making it a cost-effective method to be adopted by the pharmaceutical industry.

The novel screening systems and methods described hereinabove can also be adapted for use in screening for agents capable of at least partially reversing an abnormal cellular phenotype.

Thus, according to an additional aspect of the present invention there is provided a method of identifying agents capable of at least partially reversing an abnormal cellular phenotype.

As used herein, the term "agent" refers to a molecule(s) or a condition capable of reversing or partially reversing an abnormal cellular phenotype.

Examples of molecules which can be utilized as agents according to the present invention include, but are not limited to, nucleic acids, e.g., polynucleotides, ribozymes, and antisense molecules (including without limitation RNA, DNA, RNA/DNA hybrids, peptide nucleic acids, and polynucleotide analogs having altered backbone and/or bass structures or other chemical modifications); proteins, polypeptides, carbohydrates, lipids and "small molecule" drug candidates. "Small molecules" can be, for example, naturally occurring compounds (e.g., compounds derived from plant extracts, microbial broths, and the like) or synthetic organic or organometallic compounds having molecular weights of less than about 10,000 daltons, preferably less than about 5,000 daltons, and most preferably less than about 1,500 daltons.

Examples of conditions suitable for use as agents according to the present invention include, but are not limited to culturing conditions, such as, for example, temperature, humidity, atmospheric pressure, gas concentrations, growth media, contact surfaces, radiation exposure (such as, gamma radiation, UV radiation, X-radiation) and the presence or absence of other cells in a culture. Another condition suitable for use as an agent according to the present invention includes an infection by intracellular invading microorganisms such as , but not limited to: (i) intracellular bacteria: *Myobacterium, tuberculosis, Myobacterium leprae, Listeria monocytogenes, Brucella abortus,* (ii) intracellular fungi: *Pneumocystis carinii, Candida albicans, Histoplasma capsulatum, Cryptococcus neoformans,* (iii) intracellular parasites: *Leishmania sp.,* (iv) intracellular viruses: *Herpes simplex virus, Variola, Measles virus.*

As used herein the phrase "abnormal cellular phenotype" relates to transient or permanent deviations from normal visible or functional properties of a cell that are produced by, for example, the interaction of a genotype and the environment. An "abnormal cellular phenotype" may be associated with an aberrant expression of a cellular constituent. This cellular constituent may function as intracellular or extracellular structural elements, ligands, hormones, neurotransmitters, growth regulating factors, enzymes, chemotoxins, serum proteins, receptors, carriers for small molecular weight compounds, drugs, immunomodulators, oncogenes, second messengers, signal transducing molecules, cytokines, tumor suppressors, toxins, ions, tumor antigens, antigens, antisense inhibitors, triple strand forming inhibitors, ribozymes, or as a ligand recognizing specific structural determinants on cellular structures for the purpose of modifying their activity. As used herein, a cellular constituent associated with an abnormal cellular phenotype, can be anyone of the above mentioned gene products, or alternatively it can be a subcellular organelle or structure, examples of which are mentioned herein.

The method according to this aspect of the present invention is effected by first obtaining a cell characterized by an abnormal cellular phenotype. Cells that can be utilized by the present invention include, cell-lines, primary cultures, permanent cell cultures, preferably of mammalian origin such as but not limited to, canine, feline, ovine, porcine, equine, bovine cells, and human cells.

According to one preferred embodiment of the present invention, the cell characterized by an abnormal cellular phenotype is of a pathological origin. Examples include cancer cells, such as, for example, T47D, A431, MCF7 and SKOV3 or any other cell-line of pathological origin.

According to another preferred embodiment of this aspect of the present invention the cell characterized by an abnormal cellular phenotype is obtained via artificial intervention e.g., changing the level of expression of a gene (up or down), or the specific activity of its protein product. This may be effected by either manipulating endogenous polynucleotide sequences or by introducing exogenous polynucleotide sequences into the cell.

Manipulation of endogenous polynucleotide sequences, can be effected by, for example, introduction of cis regulatory elements. Alternatively, non-sense, mis-sense, antisense or ribozyme coding sequences can be introduced into cell in-order to specifically or non-specifically abolish transcription or translation of endogenous coding sequences.

As mentioned hereinabove an abnormal cellular phenotype can also be obtained by introducing exogenous polynucleotide sequences which encode one or more genes, or functional portions thereof into the cell. Such exogenous polynucleotides can be introduced into the cell via retroviral vectors, homologous recombination events, and the like. In some cases, the polynucleotide can be mutated, for example by random mutagenesis, point mutation at an active site, truncation, and the like, in such a way that a biological activity of the polypeptide encoded is altered.

Manipulation of endogenous polynucleotide sequences, can be effected by the use of chemicals such as but not limited to ethylmethylsulfonate, ethylnitrosourea or chemotherapeutic agents known in the art, such as Adriamycin and the like.

Alternatively, an abnormal cellular phenotype may be induced physically. Preferably, this is carried out by subjecting the cells to physical stresses such as contacting surfaces and irradiation. The latter includes gamma irradiation such as that supplied by a Cesium 137 source, etc., UV irradiation and X-irradiation. It will be appreciated that in the case of physical or chemical manipulations or a combination of the foregoing, treatment is effected over a time period sufficient to induce the desired cellular phenotype, while retaining cell viability.

Following or during generating or obtaining the cell characterized by an abnormal cellular phenotype, a detectable label is preferably coupled to a cellular constituent associated with the abnormal cellular phenotype to thereby enable close monitoring of a change in the abnormal phenotype (e.g., partial reversal).

Thus, the method according to this aspect of the present invention further includes a step of highlighting the cellular constituent. According to one embodiment highlighting can be accomplished by labeling an endogenous cellular constituent with a specific dye or a labeled antibody. Alternatively an exogenous labeled cellular constituent can be introduced within the cell. Such an exogenous constituent can be ectopically expressed from an expression construct or introduced into the cell as is. Highlighting of the exogenous cellular constituent is performed according to the selected label.

Such "labeled" cells are then subjected to the agent of interest. The agent can be either contacted with or introduced into the cell, using molecular or biochemical methodologies well known in the art. Examples include but are not limited to, transfection, conjugation, electroporation, calcium phosphate-precipitation, direct microinjection, liposome fusion and the like. Selection of a suitable introduction method is dependent upon the host cell and the type of agent used.

Following an appropriate time of incubation in the presence of the agent, cells are allowed to recover and a magnifying optical device, typically equipped with filters for detection of the reporter molecule, is used for screening for a cell in which at least a partial reversion in abnormal cellular phenotype has occurred (e.g., a change resulting in a phenotype which is closer to a normal phenotype than the abnormal phenotype).

According to another preferred embodiment of this aspect of the present invention, when the normal phenotypic pattern (e.g., level of expression, cellular distribution, biochemical modification, activity etc.) of the cellular constituent associated with the abnormal phenotype is known, such a normal pattern can be used to identify cells which exhibiting at least partial reversion of abnormal phenotype.

Alternatively, determination of reversion of an abnormal cellular phenotype is effected by comparing the pattern of the cellular constituent, associated with the abnormal cellular phenotype, following agent treatment, with the same cellular constituent when in the context of a cell characterized by a normal phenotype. This may be best illustrated by for example, actin organization in v-Src transfromed cells, versus non-transformed cells (see Example 4 of the Example section which follows).

Still alternatively, determination of reversion of an abnormal cellular phenotype is effected by comparing the pattern of the cellular constituent associated with the abnormal cellular phenotype, prior to, and following agent treatment.

Determining reversion of an abnormal cellular phenotype may also be effected by correlating two or more cellular constituents associated with an abnormal cellular phenotype. This can be effected by digital microscopy combined with cross- correlation image processing software, providing that the two cellular constituents are distinguishable. An example for such may be the distribution of epidermal growth factor receptor (EGFR) and indusial pH composition in HPV-16 E5 transformed cells (see Example #6 of the Examples section which follows).

Reversion of abnormal cellular phenotype may also be determined by correlating a cellular constituent that is associated with an abnormal cellular phenotype with a fixed cellular constituent. Such an approach is best illustrated by Example 5 of the examples section which follows.

For further confirmation of abnormal phenotype reversion, the cells are microscopically examined for specific changes, such as, for example, physiological changes including apoptosis, loss of junctions, elongation or rounding, etc.

Although the present invention can, in theory, be practiced with a single cell, such a method is not efficient nor is it desirable. Preferably, the method of the present invention is used for high throughput screening of agents using a plurality of cells to simultaneously screen a variety of agents.

In such a large scale throughput screening approach the agent may be part of a library, such as an expression library including a plurality of expression constructs each having (i) a polynucleotide encoding one of a plurality of polypeptides which is tested for an ability to reverse an abnormal phenotype (an agent), and (ii) at least one cis acting regulatory element for directing expression of the polypeptides from the expression construct.

Alternatively, chemical libraries available, for example, from chemical companies including Merck, Glaxo, Novartis, and Bristol Meyers Squib can also be utilized for screening. Optionally, libraries of natural compounds in the form of bacterial, fungal, plant and animal extracts, which are available from, for example, Pan Laboratories or Mycosearch or are readily producible by methods known in the art can also be utilized by the present invention.

When performing large-scale screening, the cell population that is subjected to individual agents can be compartmentalized so as to facilitate identification of abnormal phenotype reversal. This may be effected by alliquoting the cell population into flat glass-bottom multiwell plates at a pre-calibrated density which allows the growth of just one or two clones per well.

In the step of screening a manual procedure can be followed, although automated screening using robots, such as multiwell attachment for the Delta Vision microscope, Cellomics automated microscope, are preferred.

Once identified, agents capable of at least partially reversing an abnormal cellular phenotype are recovered. If the agent is a polynucleotide or a polynucleotide expression product, cells are isolated and propagated and are used for isolating the polynucleotides agents, by, for example, PCR amplification, as discussed above.

The retrieved agents are further analyzed for their exact mechanism of action and adjusted for optimal effect, using various biochemical and cell-biology methods. Eventually, distinguishing which of the agent isolated is a potential a lead compound can be accomplished by testing the effect of the agent in pharmacological models of various diseases. Agents that affect disease progression or onset, constitute leads for drug development.

Such agents can be applied for treatment of many pathological states such as cancer, metabolic disorders such as, diabetes and obesity, cardiopulmonary diseases, viral infections and other known syndromes and diseases.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshals et al., "Strategies for Protein Purification and Characterization - A Laboratory Course Manual" CSHL Press (1996); all of which are incorpotaed by reference as if fully set forth herein.

## EXAMPLE 1

### Co-localization analysis of pairs of proteins in cells

To determine the spatial relationships between different proteins in cells, images of the two labels were acquired and subjected to correlation analysis (according to Eq. 1, where N and M are the images of the two labels).

Figure 1 illustrates 10 examples for such comparison. It is shown that for largely overlapping structures (i.e., cadherin/catenin; tensin/a5; paxillin/PY) high values (> ~0.7-0.8) were obtained. No correlation (i.e., cadherin or catenin/ DAPI) were close to zero. Partial overlaps gave intermediate values.

## EXAMPLE 2

### Effect of actomyosin inhibitors on tensin motility in live cells

Inhibitors of acto-myosin contractility, like latrunculin-A (Lat-A), H-7 and ML-7, block the translocation of fibrilar adhesion (Zamir et al., Nature Cell Biology, Vol. 2, 191-196, 2000). In order to follow the kinetics of this effect a correlation analysis according to Eq. 1 was applied on digital movies of GFP-tensin transfected cells before, during and after treatments with inhibitors. Before treatment, fibrilar adhesions were highly dynamic and thus the correlation between two sequential frames was low (<0.65, Figure 2). Following the addition of Lat-A, H-7 or ML-7 the translocation of fibrillar adhesion was inhibited, leading to higher correlation between sequential frames (~0.78, 0.81 and 0.92 for Lat-A, H-7 and ML-7, respectively). The duration till maximum respond to Lat-A was relatively short (~18 minutes after addition), while the respond to H-7 and ML-7 were more gradual (~50 minutes after H-7 addition and ~80 minutes after ML-7 addition). After washing-cut the drugs, the translocation of fibrillar adhesion recovered and the correlation dropped back to lower levels. The recovery from H-7 was relatively fast (<20 minutes) while recovery from Lat-A and ML-7 took relatively long time (>80 minutes).

### EXAMPLE 3

### *Identifying distortions between images of the same field*

To examine the ability of correlation analysis to characterize distortions between images a rotated, translated or shrunken variants from one original image were created, and two composites were made: one from 4 original images and a second from the original, translated, contracted and rotated variants (Figure. 3). The composite images were divides to 784 square frames and for each frame the translation vector that maximizes the correlation between the two images (Eq. 4, [$a^{max}$, $b^{max}$]) was found. The results found for each frame are displayed by a line with size and direction equal to the translation vector and a color proportional to the maximal correlation found (Eq. 5, $C^{max}$). The results indicate that the translation vectors indeed tracked successfully the local translation of structures, and, as a whole, indicated the type of global distortion performed (contraction, rotation, translation and not-distorted) in the four sub-domains of the composite. Since the search for maximal correlation is limited to translational shifts, a correlation of 1 was found only for the motionless and translated sub-domains (Figure 3).

### EXAMPLE 4

### *Loss of actin cytoskeleton in H-Ras transformed cells*

Background:

Cancer cells are often characterized by an altered morphology, exhibiting poor adhesions to neighboring cells or to the extracellular matrix and a disorganized cytoskeleton (17). These changes are believed to be responsible for some of the malignant properties of cancer cells, including their enhanced motility, loss of contact inhibition and anchorage dependence as well as their tendency to dissociate from their local sites and either invade neighboring tissues or form distant metastatic foci. Altered morphology or "transformed phenotype" is a feature of primary and secondary tumor cells in-vivo, but can be also appreciated in culture (18). One of the most prominent features of transfonnation is the loss of organized actin cytoskeleton, which has been reported for a large variety of tumor cells in-vivo and oncogene transformed cells in-vitro. In both cases loss of stress fibers is noted, with a parallel reduction in matrix-attached focal adhesions. Thus, a screen aimed at isolation of pharmacological agents which reverse transfonned phenotype originating from oncogene infection can be effected by the present invention.

H-Ras infected GFP-actin reporter cell line: Reporter cells of rat or human origin expressing actin fused to a green fluorescent protein (GFP) can be infected with a retroviral vector encoding H-Ras to induce cell transformation.

Screening and analysis: Transformed cells can be cultured in multiwell plates and treated with pharmaceutical agents for time intervals which allow cytoskeletal changes. Subsequently, treated cells can be examined directly for actin levels, using filament detection and quantification algorithms. Agents that induce reversion of the transformed phenotype can be identified and further characterized.

### EXAMPLE 5

### *Down regulation of MHC-1 by HIV-1 Nefprotein*

Background:

The human and simian immunodeficiency viruses (HIV and SIV) are able to down-regulate the expression of the major histocompatibility complex type I (MHC-I), a critical mediator of immune recognition on the surface of the host cell (19). For this down-regulation, HIV employs three different mechanisms mediated by three different viral proteins. The viral Tat protein represses transcription of the MHC-I, Vpu retains nascent MHC-I chains in the endoplasmic reticulum and Nef mediates selective internalization of MHC-I molecules from the plasma membrane. These mechanisms of MHC-I down-regulation allow HIV infected cells to avoid detection by cytotoxic T lymphocytes (CTL) (20). However, class I down regulation potentially exposes the virus-infected cell to an attack by natural killer (NK) cells. This discrepancy can be explained by the fact that HIV selectively down regulates specific components of the MHC-I. While HLA-A and HLA-B are significantly down regulated by HIV, no effect is observed on HLA-C or HLA-E, which protect human lymphoid cells from NK cell cytotoxicity. This selective down regulation allows HIV-infected cells to avoid NK cell-mediated lysis and may represent (for HIV) a balance between escape from CTL and maintenance of protection from NK cells (21). Understanding the molecular mechanism enabling HIV infected cells to avoid recognition by the immune system, can serve a basis for isolating therapeutic agents.

Nef infected reporter cell: Human lymphoid cells are infected with a mammalian expression vector, which directs the expression of HIV Nef protein. Control cells infected with the vector alone serve as a control.

Screening and analysis: Infected cells can be cultured in multiwell plates and treated with pharmaceutical agents for time intervals which allow up-regulation of the MHC-I. Specifically, cells can be extracellularly stained, with a fluorophore-conjugated antibody directed at HLA-A. Agents that are recognized positive in this screen, namely agents, which up regulate HLA-A, can be further analyzed as potential therapeutic agents.

### EXAMPLE 6

### Altered endosomal acidification in HPV-16 E5 infected cells

Background:

Human papillomaviruses (HPVS) infect basal human keratinocytes and propagate in the differentiating layers of the epithelium (22). The E6, E7 and E5 proteins of certain types of HPVs, possess oncogenic activities that contribute to the pathogenesis associated with HPV infection (23,24). In particular, the HPV-16 E5 open reading frame encodes a small, highly hydrophobic protein (25) with activities that may contribute both to the pathogenicity of the virus and to its replication. This protein possesses mitogenic activity that act synergistically with epidermal growth factor (EGF) in human keratinocytes and inhibits the degradation of the EGF receptor (EGFR) in endosomal compartments following ligand-mediated receptor endocytosis (26). Indeed, in E5-infected keratinocytes, a significant inhibition of endosomal acidification is observed, which may explain the prolonged retention of undegraded EGFR molecules in intracellular vesicles. Inhibition of endosomal acidification is mediated through the binding of E5 to the 16-kDa subunit of the vacuoiar-proton-ATPase (27). This pump establishes and maintains the low internal pH (4.5 to 5.0) in endosomes and lysosomes relative to the cytoplasmic pH (7.0 to 72) by utilizing the energy from ATP hydrolysis to generate an influx of protons into the vesicle (28). Thus, organelle acidification, which correlates with HPV replication efficiency can serve as a tool for screening for potential therapeutic agents to a range of malignant carcinomas.

Generation of HPV-16 E5 reporter cells: Human foreskin keratinocytes, which serve as normal host cells for HPV, can be transfected via electroporation with an E5 encoding gene under the control of a suitable promoter. Transfected clones are resolved for E5 expression by westem blot analysis and endosomal pH is determined using lysosensor green DND-189 (hrtp://www.probes.com/handbook/chapter 21.3).

Screening and analysis: Transformed cells can be cultured in multiwell plates and treated with pharmaceutical agents. Subsequently, treated cells can be applied with lysosensor green (described hereinabove) as indicated by the manufacturer (Molecular Probes Inc.). Agents that induce reversion of the transformed phenotype can be visualized by normal acidification of vesicular pH, using digital microscopy.

### EXAMPLE 7

### Upregulation of Phospholipase D (PLD) activity in multidrug resistance (MDR) tumor cells

Background:

Multidrug resistance (MDR) is a major cause of failure of cancer chemotherapy and is often associated with elevated expression of drug transporters such as P-glycoprotein (P-gp) in the cancer cells (29). A variety of stimuli increase the expression of the mdr1 gene: lowered extracellular pH, heat shock, arsenite, cytotoxic agents, anticancer drugs, transfections with oncogenes, HIV-I and UV-irradiation. MDR is, however, accompanied by additional biochemical changes including modifications of membrane composition and properties, such as massive upregulation of caveolin expression and an elevated surface density of caveolae. Furthermore, phospholipase D (PLD), a constituent enzyme of caveolae and detergent-insoluble glycolipid-rich membrane (DIGs), is up-regulated in human MDR cancer cells (30). Thus, agents directed at reversing the MDR phenotype are of great importance to the field of cancer therapy.

GFP-caveolin-1 transfected MDR reporter cells- MCF7-AdrR human breast cancer cells can be selected as model MDR cells. These cells along with control cells not bearing the MDR phenotype can be transfected with GFP-caveolin-1.

Screening and analysis- Transfected cells can be cultured in multiwell plates and treated with pharmaceutical agents for time intervals which allow down regulation of caveolin-1. Subsequently, caveolin-1 levels of treated cells are determined. Agents that induce reversion of the MDR phenotype can be identified and further characterized.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. In addition, particular details and examples provided in context of any of the aspects of the present invention, yet are not specifically described in context of other aspects of the invention, are meant to be available and descriptive also in context of any such other aspects of the invention.

REFERENCES

(Addition1 references are cited in the text)

(1) Burbaum, J., (1998) Drug. Discov. Today. 3:313-322

(2) Yates JR. (2000) Trends Genet. 16:5-8

(c) Iwabuchi K. (1993) Oncogene. 8:1693-1696

(4) Fashena SJ. et al. (2000) Gene. 250: 1-14

(5) Andersen JS. (1997) Mass Spec. Rev.

(6) Jensen ON . (1999) Methods Mol. Biol. 112:513-30

(7) Hill CM (1994) J. Immunol. 152:2890-2898

(8) Worm D (1996) Diabetologia 39:142-148

(9) Sundberg SA. (2000) Curr. Opin. Biotech. 11:47-53

(10) Zamir E. (2000) Nature Cell Biol. 2:191-196

(11) Misawa (2000) PNAS 97:3062-3066

(12) Ojcius C. Res. Immunol. (1996) 177(3):175-88

(13) Uchimiya, H. (1982) Cong. Plant Tissue and Cell Culture, Jap. Assoc. for Plant Tissue Culture, Tokyo 507-50

(14) Piacumakos EG., (1973) Methods Cell Biol. 287-311

(15) Yamamoto F. (1982) Exp. Cell Res. 142:79-84

(16) Purres RD. (1981) Acad. Press NY. 146

(17) Button. E. (1995) Cell. Motil. Cytoskeleton 30:247-251

(18) Janmey PA. and Chaponnier C. (1995) Curr. Opin. Cell. Biol. 1:111-117

(19) Scheppler JA. (1989) J. Immunol. 143:2858-66

(20) Kamp W. (2000) Eur. J. Clin. Invest. 30:740-746

(21) Cohen GB. (1999) Immunity 10:661-671

(22) McCane DJ. (1994) Infect. Dis. Clin.N. Am. 8:751-767

(23) Chesters PM. (1990) J. Gen. Virol. 71:449-453

(24) Leptak, CS. (1991) J. Virol. 65:7078-7083

(25) Bubb VD. (1988) Virology 163: 243-246

(26) Straight SW. (1993) J. Virol. 67:4521-4532

(27) Straight SW. (1995) J. Virol. 69:3185-3192

(28) Gollaway CJ. (1988) Methods. Enzymol. 157:601-611

(29) Krishna R. and Mayer LD. (2000) Eur. J. Pharm. Sci. 11:265-283

(30) Fiucci G. (2000) Int. J. Cancer 85:882-888

**Claims**

1.  A method of identifying at least one agent capable of at least partially reversing an abnormal cellular phenotype, the method comprising:

    (a) exposing the cell **characterized by** the abnormal phenotype to the at least one agent; and
    (b) monitoring a change in at least one cellular constituent being associated with the abnormal cellular phenotype, said change being indicative of at least a partial phenotype reversal, to thereby determine the capability of the at least one agent in at least partially reversing the abnormal cellular phenotype.

2.  The method of claim 1, further comprising a step a highlighting said at least one cellular constituent, wherein said step of highlighting is effected by at least one method selected from the group consisting of:

(i) labeling said at least one cellular constituent being associated with the abnormal cellular phenotype;

(ii) providing said at least one cellular constituent being associated with the abnormal cellular phenotype within said cell, wherein said at least one cellular constituent being fused to a label.

3. The method of claim 2, wherein said providing said at least one cellular constituent is effected by introducing into the cell said at least one cellular constituent or a nucleic acid construct for expressing said at least one cellular constituent.

4. The method of claim 2, wherein said label is selected from the group consisting of a fluorescent label, a radioactive label, an epitope label, a biotin label and an enzyme label.

5. The method of claim 1, wherein said change in said at least one cellular constituent includes a change in an at least one parameter selected from the group consisting of a cellular distribution, a biochemical modification, an expression level and an activity of said at least one cellular constituent.

6. The method of claim 1, wherein said step of monitoring a change in said at least one cellular constituent is effected by at least one method selected from the group consisting of:

(i) comparing said at least one cellular constituent of said cell with that of a second cell, **characterized by** a normal cell phenotype; and

(ii) comparing said at least one cellular constituent within said cell prior to step (a), and following step (b).

7. The method of claim 1, wherein said cell is of a mammalian origin.

8. The method of claim 1, wherein said at least one agent is selected from the group consisting of a test condition and a test compound.

9. The method of claim 8, wherein said test condition is selected from the group consisting of a growth condition and a radiation condition.

10. The method of claim 8, wherein said test compound is selected from the group consisting of a synthetic product and a natural product.

11. The method of claim 1, wherein said at least one cellular constituent is selected from the group consisting of a protein, a lipid, a second messenger, an ion, a free radical, a subcellular organelle and a structure.

12. The method of claim 1, further comprising a step of generating said cell **characterized by** the abnormal cellular phenotype, prior to step (a).

13. The method of claim 1, wherein said cell **characterized by** the abnormal cellular phenotype is of a pathological origin.

14. The method of claim 1, further comprising a step of fixing said cell prior to step (c).

15. A method of quantifying a co-localization of a plurality of distinguishable molecules in a cell, the method comprising the steps of:

(a) acquiring an image of the cell for each of said plurality of distinguishable molecules so as to obtain a plurality of images of said cell, each individual image of said plurality of images presenting a distribution of one of said plurality of distinguishable molecules in the cell; and

(b) calculating a correlation coefficient for at least one pair of said individual images, thereby quantifying the co-localization of at least a pair of said distinguishable molecules in the cell.

16. The method of claim 15, wherein each of said plurality of images is a normalized image.

correlation

cadherin
β-catenin                    0.86

cadherin
DNA                         -0.56

β-catenin
DNA                         -0.45

tensin
α₅                           0.85

paxillin
PY                           0.87

Fig. 1

Fig. 1 (Continued)

Fig. 2

Fig. 3